# EUROPEAN PATENT SPECIFICATION

(11) **EP 2 005 886 B1**
(45) Date of publication and mention of the grant of the patent: **14.09.2011**
(21) Application number: 07252483.8
(22) Date of filing: 19.06.2007
(51) Int. Cl.: A61B 5/103

(54) **Method and apparatus for measuring skin texture**
Verfahren und Vorrichtung zum Messen der Hauttextur
Procédé et appareil de mesure de la texture de la peau

(43) Date of publication of application: 24.12.2008
(73) Proprietor: Biocompatibles UK Limited, Farnham Surrey GU9 8QL (GB)
(72) Inventor: Symon Cotton, SG19 3QS (GB); Rober Morse, CB1 3PU (GB); Mark Chellingworth, Vale Of Glamorgan Wales CF64 2NP (GB)
(74) Representative: Dawson, Elizabeth Ann

(56) References cited:
- EP-A- 1 768 060
- US-A1- 2001 056 237
- US-A1- 2005 030 372
- US-A1- 2006 274 921
- US-A1- 2006 276 966
- US-B1- 6 208 749
- DATABASE INSPEC [Online] THE INSTITUTION OF ELECTRICAL ENGINEERS, STEVENAGE, GB; April 2006 (2006-04), DE ROODE R ET AL: "Multispectral system evaluates treatments in dermatology" XP002463296 Database accession no. 8954425 & Laser Focus World PennWell Publishing USA, vol. 42, no. 4, April 2006 (2006-04), pages 102-104, ISSN: 1043-8092
- RAMELLA-ROMAN J C ET AL: "Out-of-plane polarimetric imaging of skin: surface and subsurface effects" PROCEEDINGS OF THE SPIE, SPIE, BELLINGHAM, VA, US, vol. 5686, no. 1, April 2005 (2005-04), pages 142-153, XP002386128 ISSN: 0277-786X

## Description

The present application relates to methods and apparatus for measuring skin texture. In particular embodiments of the present application concern methods and apparatus for measuring skin texture and identifying areas of dry skin.

When the skin is viewed in close up the surface is composed of fine lines and wrinkles. Detailed measurements of these structures are of great interest in both the research of products designed to reduce the appearance of wrinkles and also in the education of consumers. Standard techniques to measure the topology of skin range from making physical silicon replicas of the skin which are than traced to stereo and fringe projection. All of these techniques produce excellent results but are limited to laboratory analysis due to costs and acquisition times. An alternative system for measuring the detailed topology of the skin is therefore desirable.

US2006/274921 provides for methods and systems for performing a biometric function. A purported skin site of an individual is illuminated with illumination light. The purported skin site is in contact with a surface. Light scattered from the purported skin site is received in a plane that includes the surface. An image is formed from the received light. An image-texture measure is generated from the image. The generated image-texture measure is analyzed to perform the biometric function.

US6208749 provides for systems and methods for the multispectral imaging of skin tissue enables automatic characterization of the condition of a region of interest of the skin, based on direct digital imaging of the region of interest or the digitization of color photographic slides of the region of interest, illuminated by appropriately filtered light. Preferably, a digital image at a low spectral band is automatically segmented and that segmented mask is used to segment the other images by a digital processor. Parameters related to the texture, asymmetry, blotchiness and border irregularities are also automatically estimated. The region of interest is automatically characterized by the digital processor, based on those parameters. The region of interest may include a skin lesion, in which case the present invention enables the characterization of the lesion as malignant or benign.

US2001/056237 provides for an apparatus for monitoring the presence of one or more chromophores in a tissue sample, comprises a light source for projecting light to illuminate an area of such tissue sample, a photo-receptor for receiving light remitted by the illuminated area of tissue, and spectroscopic analyser means for monitoring the remitted light, a comparator having means for comparing variations in the intensity and spectral characteristics of the remitted light with respect to the intensity and spectral characteristics of the projected light at different wavelengths and with a record of the intensity and spectral characteristics of light remitted by a reference sample of such tissue and means for emitting a control signal in response to any such variations. Methods of analyzing tissue histology, especially skin histology, are discussed, and a mathematical model is proposed for the analysis and comparison of the remitted light with a reference sample.

In accordance with one aspect of the present invention there is provided a method of measuring skin surface texture comprising:
illuminating an area of skin with polarized light;
obtaining a measurement of light returned by the illuminated area of skin in a first and a second waveband, wherein the measured light in the first waveband is light having a different polarity to the light with which said area of skin is illuminated and the measured light in the second waveband comprises light having the same and different polarities of light as the light with which said area of skin is illuminated;
processing the measurement of light in the first waveband to determine an estimated expected level of light in said second waveband returned by the illuminated area of skin utilising a model of the interaction of light with at least one chromophore in the skin; and
determining a measurement of the surface texture of the imaged illuminated area of skin on the basis of the difference between the estimated and actual levels of light in said second waveband returned by the illuminated area of skin.

In accordance with a further aspect of the present invention there is provided an apparatus for measuring skin surface texture, the apparatus comprising:
a light source operable to illuminate an area of skin with polarized light;
a detector operable to:
   obtain a measurement of light returned by an illuminated area of skin in a first waveband and a second waveband, wherein the measured light in the first waveband is light having a different polarity to the light with which said area of skin is illuminated by said light source and the measured light in the second waveband comprises light having the same and different polarities of light as the light with which said area of skin is illuminated by said light source; and
a processor operable to:
   process an obtained measurement of light in a first waveband to determine an estimated expected level of light in a second waveband returned by an illuminated area of skin utilising a model of the interaction of light with at least one chromophore in the skin; and
   determine a measurement of the surface texture of an imaged illuminated area of skin on the basis of the difference between estimated and obtained actual levels of light in said second waveband returned by an illuminated area of skin.

Further aspects and embodiments of the present invention will become apparent with reference to the accompanying drawings in which:
Figure 1 is a schematic cross sectional view through a layer of skin illustrating the structure of the skin and the interaction of that structure with incident light;
Figure 2 is a schematic block diagram of a skin texture measurement system in accordance with an embodiment of the present invention;
Figure 3 is a flow diagram of the processing performed by the skin texture measurement system of Figure 2;
Figure 4 is a graph illustrating the relationship between the reflection of red and infra-red light by skin with a fixed amount of collagen;

### Interaction of Light with the Skin

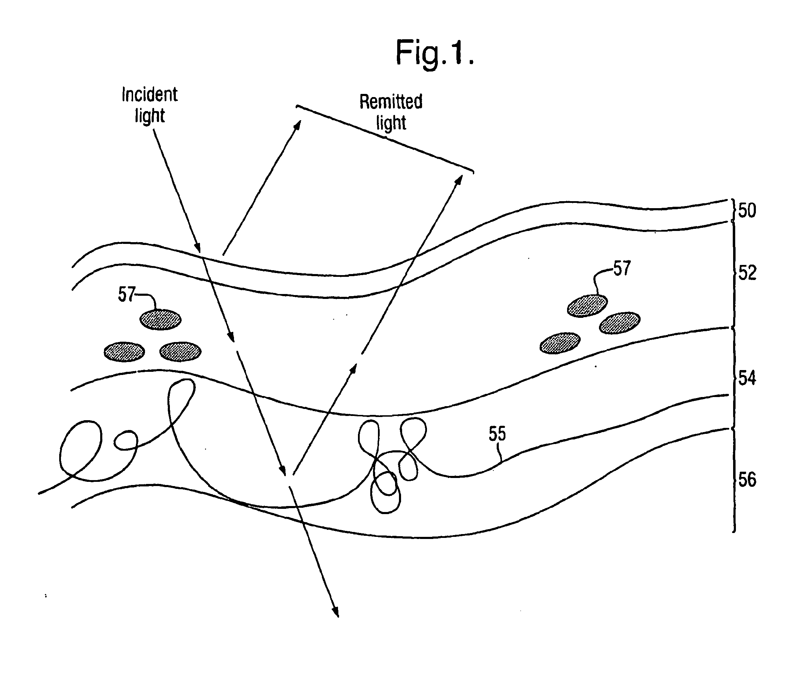

By way of background and to assist understanding, before describing embodiments of the present invention, the physical structure of skin and the interaction of skin with light will first be briefly explained with reference to Figure 1.

As shown in Figure 1, skin has a layered structure comprising an outer cornified layer 50 also known as the stratum corneum, the epidermis 52, and the dermis which itself can be divided into the papillary dermis 54 which contains the blood supply 55 for the skin and the reticular dermis 56.

When light is incident on the skin, much of the light is immediately reflected when coming into contact with the outer cornified layer 50. A proportion of incident light does, however, pass through the cornified layer 50 and proceeds to interact with the constituents of the epidermis 52 and the papillary dermis 54. As light passes through the epidermis 52 and the papillary dermis 54 the light is absorbed by various chromophores present in the skin, most notably chromophores such as haemoglobin present in the blood in blood vessels 55 in the papillary dermis, melanin, a pigment produced by melanocytes 57 in the epidermis 52 and collagen a fibrous material present throughout the skin. By the time the incident light reaches the reticular dermis 56 the scattering of light is highly forward and therefore for that reason the reticular dermis 56 can for all intents and purposes be considered returning no light.

In addition to chromophores present in the epidermis 52 and papillary dermis 54 absorbing various wavelengths, certain structures in the skin most notably collagen cause incident light to be reflected. The outward appearance of the skin can therefore be considered to be a mixture of the light immediately reflected by the cornified layer 50 and the remitted light which has interacted with the chromophores present in the epidermis 52 and the papillary dermis 54.

As will be described, the present invention utilises the fact that the appearance of the skin is dependent upon the reflection of light from the surface of the skin and the interaction of light with structures and chromophores below the surface to obtain a measurement of the skin's surface texture.

### Specific Embodiment

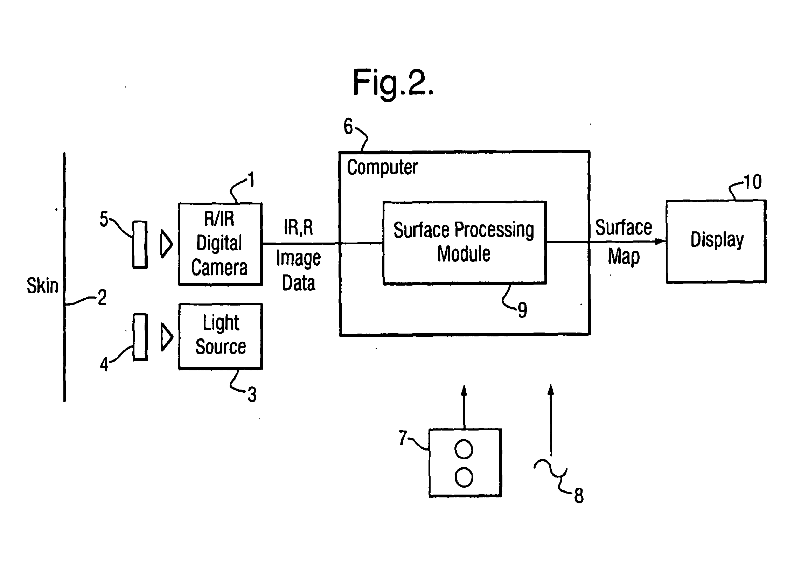

An embodiment of the present invention which now be described with reference to Figures 2 - 4.

Referring to Figure 2 which is a schematic block diagram of an embodiment of the present invention, a digital camera 1 comprising a digital camera operable to obtain red and infra-red images of light with wavelengths of approximately 650nm and 900nm respectively is provided which is arranged to obtain an image of the surface of the skin of an individual 2 illuminated by a light source 3.

Provided in front of the lens of the digital camera 1 and the light source 3 are a first 4 and a second polarizer 5. These polarizers 4,5 are conventional polarizers which polarize visible light having wavelengths in the range of 400 to 700 nm with the second polarizer 5 being arranged so as to be cross polarized with the first 3.

The interaction of light with collagen in the skin is such to cause the light to loose its original polarization. Light detected by the red detectors of the digital camera 1 when an area of skin 2 is illumined by the light source 3 via the first polarizer 4 therefore comprises red light which has passed through the surface of the skin and interacted with the chromophores and collagen in the skin below the surface. This is because the polarized red light directly reflected from the surface of the skin will be filtered by the cross polarization of the second polarizer 5 in front of the lens of the digital camera 1.

In contrast, light detected by the infra-red detectors of the digital camera 1 when an area of skin 2 is illuminated by the light source 3 via the first polarizer 4 will pass through the second polarizer 5 regardless of whether the light has had its polarization altered through interaction with collagen in the skin since the range of the polarizers 4,5 does not extend to infra-red light. The infra-red light detected by the digital camera 1 will therefore comprise a mixture of infra-red light which has been reflected directly from the surface of the skin 2 infra-red light which has interacted with the chromophores and structures of the skin 2 below the surface.

The red and infra-red images obtained by the digital camera 1 are then transmitted to a computer 6 which is configured by software either provided on a disk 7 or by receiving an electrical signal 8 by via a communications network to be configured to include a surface processing module 9 to process the image data in the manner described below to generate a surface map illustrating the detailed variations in the surface of the skin 2 imaged by the camera 1. This surface map is then shown on a display 10.

### Processing of Obtained Image Data

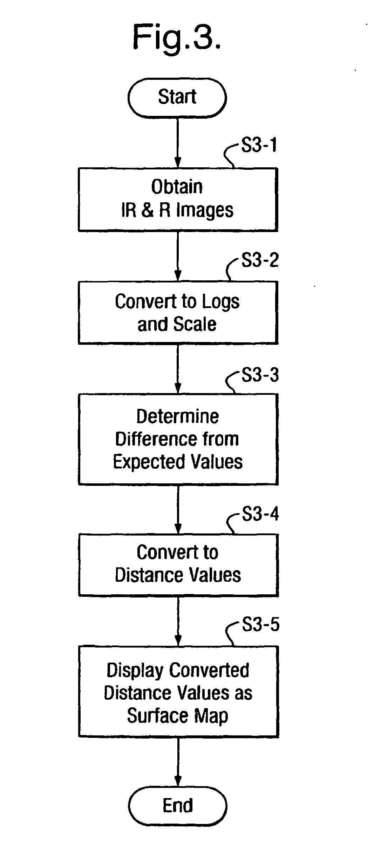

Referring to Figure 3 which is a flow diagram of the processing performed by the computer 6 of Figure 2, initially (S3-1) an image is obtained by the digital camera 1 of the area of skin 2 illuminated by the light source 3.

In this embodiment image data generated by the digital camera 1 comprises R and IR values ranging from 0 to 255 for a large array of pixels where the R and IR values are indicative of the extent light received by a photo receptor within the camera 1 for each pixel in an image appears to be red or infra-red where a completely cold black pixel has R and IR values of 0, 0 and a completely hot bright white pixel has R and IR values of 255, 255.

When an image of an area of skin 2 has been obtained by the camera 1, the surface processing module 9 then proceeds to process (S3-2-S3-4) each pair of R, IR pixel values in the obtained image in turn to convert the R, IR pixel values into values indicative of surface texture.

In this embodiment, this conversion is based upon two of assumptions.

Firstly, it is assumed that the skin surface 2 is substantially flat and the illumination of the skin surface is substantially uniform. This will be the case where a small area of skin in being imaged and it is possible to bring the light source 3 and camera 1 into close proximity of the skin 2 being analysed.

Secondly, it is assumed that the area of skin is a healthy area of skin with uniform a thickness of collagen of 0.2mm.

Under such circumstance, the ratio of the red and infra-red light detected can be considered as only affected by variations in concentrations of melanin and small scale variations in the surface of the skin the since both red and infra-red light is substantially unaffected by the presence of haemoglobin.

In this embodiment, natural logarithms of the R and IR values for a pixel are first taken and then the resultant logarithms are scaled so as to fall been a minimum value of 0 and a maximum value of 1 (S3-2). The difference between the actual scaled logarithm of the detected infra-red value IR is then compared (S3-3) with an expected infra-red value derived from the scaled logarithm of the detected red value R.

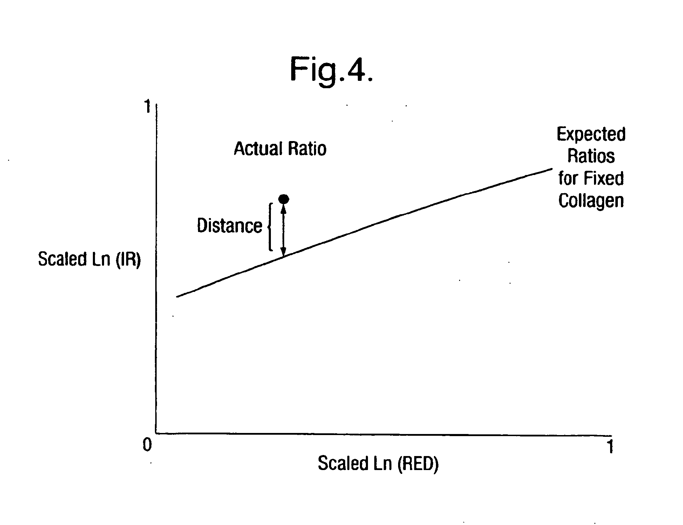

Figure 4 is a graph illustrating the relationship between the reflection of red and infra-red light by skin with a fixed amount of collagen in the absence of any surface reflection. In such circumstance the ratio of light is entirely dependent upon the concentration of melanin present within the epidermis which can be considered to be a perfect exponential term. In the graph of graph of Figure 4 where the axes are scaled logarithmic axes, this means that expected ratios of red and infra-red values fall on a straight line. The difference between an expected infra-red value and the actual value derived by scaling the logarithm of the IR value for a pixel arises due to the occurrence of surface reflection. A measurement of the surface texture at a point corresponding to a pixel in an obtained image can then be obtained (S3-4) by taking the antilog of the calculated distance between the actual infra-red value and the expected infra-red value determined from the detected level of reflected red light.

This process (S3-2-S3-4) is then repeated for all of the pixels in the obtained images and the resultant converted difference values are then displayed (s3-5) as a surface map.

In generating the surface map, although the assumption that the thickness of collagen is uniform is not likely to be true, variations in converted distance due to the usual variation in collagen thickness within the range of normal skin are significantly smaller than the variations arising due to variations arising to differences in surface reflection to differences in the surface topology of the skin and hence do not have an appreciable impact on the accuracy of the obtained measurements.

In the resultant surface map, pixels where little or non-surface reflection has occurred which will correspond to wrinkles or furrows in the skin will be associated with lower values with the relative size of the measurement indicative of the depth of the furrow or wrinkle. Additionally, the obtained map can also be used to measure the extent of areas of dry skin as such areas are associated with higher converted distance values and areas of surface maps indicative of more alpine skin topology.

### Alternative Embodiments and Modifications

Although in the above described embodiment a skin texture analysis system has been described which processes red and infra-red images, alternative systems could be used.

Thus for example, instead of a red/infra-red digital camera, a conventional RGB camera could be utilised. In such an alternative embodiment polarizers would have to be provided which did not extend through the entire range of detection of the camera so that at least one image could be obtained which was an image based on a mixture of light directly reflected from the surface of the skin and light which interacts with the structures and chromophores in the skin.

Although in the above embodiment a measure of skin texture is obtained using two images of the skin more images could be utilised. More specifically, in the above embodiment red and infra-red images are processed to obtain a skin surface measurement. Utilising red and infra-red images is preferable because light of these wavelengths is substantially unaffected by the presence of haemoglobin. In other embodiments an additional colour image, for example one based on green light could be obtained. The detected levels of green and red light could then be utilised to determine estimates of both blood and melanin concentrations present in the skin. The expected levels of infra-red light based on the determined concentrations could then be compared with the actual detected levels to determine a measurement of surface texture.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier can be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means.

When a program is embodied in a signal which may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

## Claims

1. A method of measuring skin surface texture comprising:
illuminating an area of skin (2) with polarized light;
obtaining (S3-1) image data representing a measurement of light returned by the illuminated area of skin (2) in a first and a second waveband, wherein the measured light in the first waveband is light having a different polarity to the light with which said area of skin (2) is illuminated and the measured light in the second waveband comprises light having the same and different polarities of light as the light with which said area of skin (2) is illuminated;
processing (S3-2-S3-3) image data representing the measurement of light in the first waveband to determine an estimated expected level of light in said second waveband returned by the illuminated area of skin (2) utilising a model of the interaction of light with at least one chromophore in the skin; and
determining (S3-4) a measurement of the surface texture of the imaged illuminated area of skin (2) on the basis of the difference between the estimated and actual levels of light in said second waveband returned by the illuminated area of skin (2).

2. A method in accordance with claim 1 wherein said first waveband comprises a waveband corresponding to visible light.

3. A method in accordance with claim 2 wherein said first waveband comprises a waveband corresponding to red light.

4. A method in accordance with claim 1, 2 or 3 wherein said second waveband comprises a waveband corresponding to infra-red light.

5. A method in accordance with any preceding claim wherein said at least one chromophore in the skin comprises melanin.

6. A method in accordance with any preceding claim, further comprising:
obtaining (S3-1) image data representing a measurement of light returned by the illuminated area of skin (2) in a third waveband, wherein the measured light in the third waveband is light having a different polarity to the light with which said area of skin (2) is illuminated;
wherein processing (S3-2-S3-3) image data representing the first measurement of light to determine an estimated level of light in said second waveband returned by the illuminated area of skin (2) comprises:
processing (S3-2-S3-3) image data representing the measurements of light in said first and third wavebands to determine an estimated expected level of light in said second waveband returned by the illuminated area of skin utilising a model of the interaction of light with a first and a second chromophore in the skin.

7. A method in accordance with claim 6, wherein said first and second chromophore comprise melanin and haemoglobin.

8. An apparatus for measuring skin surface texture, the apparatus comprising:
a light source (3,4) operable to illuminate an area of skin (2) with polarized light;
a detector (1,5) operable to:
obtain image data representing a measurement of light returned by an illuminated area of skin (2) in a first waveband and a second waveband, wherein the measured light in the first waveband is light having a different polarity to the light with which said area of skin (2) is illuminated by said light source (3,4), and the measured light in the second waveband comprises light having the same and different polarities of light as the light with which said area of skin is illuminated by said light source (3,4); and
a processor (6,9) operable to:
process obtained image data representing a measurement of light in a first waveband to determine an estimated expected level of light in a second waveband returned by an illuminated area of skin utilising a model of the interaction of light with at least one chromophore in the skin; and
determine a measurement of the surface texture of an imaged illuminated area of skin (2) on the basis of the difference between estimated and obtained actual levels of light in said second waveband returned by an illuminated area of skin (2).

9. An apparatus in accordance with claim 8 wherein said light source (3,4), operable to illuminate an area of skin (2) with polarized light comprises a light source (3) operable to illuminate an area of skin (2) via a polarizing filter (4).

10. An apparatus in accordance with claim 8 or 9 wherein said detector (1,5) comprises:
a digital camera (1) operable to obtain an image of an illuminated area of skin (2) via a polarizing filter (5) wherein the polarizing filter (5) is operable to polarize light in said first waveband without polarizing light in said second waveband.

11. An apparatus in accordance with claim 8, 9 or 10 wherein said first waveband comprises a waveband corresponding to visible light.

12. An apparatus in accordance with claim 11 wherein said first waveband comprises a waveband corresponding to red light.

13. An apparatus in accordance with any of claims 8 - 12 wherein said second waveband comprises a waveband corresponding to infra-red light.

14. An apparatus in accordance with any of claims 8 - 13, wherein said processor (6,9) is operable to:
process an obtained measurement of light in a first waveband to determine an expected value of light in said second waveband returned by the illuminated area of skin (2) utilising a model of the interaction of light with melanin in the skin.

15. An apparatus in accordance with any of claims 8 - 12, wherein said detector (1,5) is operable to obtain image data representing a measurement of light in a third waveband returned by an illuminated area of skin (2), wherein the measured light in the third waveband is light having a different polarity to the light with which said area of skin (2) is illuminated; and
wherein said processor (6,9) is operable to:
process obtained image data representing measurements of light in said first and third wavebands to determine an estimated expected level of light in said second waveband returned by an illuminated area of skin utilising a model of the interaction of light with a first and a second chromophore in the skin.

16. An apparatus in accordance with claim 15, wherein said first and second chromophore comprise melanin and haemoglobin.

17. A recording medium (7,8) storing computer interpretable instructions for causing a programmable computer (6) to be configured to receive image data representing a measurement of light returned by an illuminated area of skin (2) in a first and a second waveband obtained by a detector (1,5), wherein the measured light in the first waveband is light having a different polarity to the light with which said area of skin (2) is illuminated and the measured light in the second waveband comprises light having the same and different polarities of light as the light with which said area of skin (2) is illuminated to:
process (S3-2-S3-3) the received image data representing a measurement of light in the first waveband to determine an estimated expected level of light in said second waveband returned by the illuminated area of skin (2) utilising a model of the interaction of light with at least one chromophore in the skin; and
determine (S3-4) a measurement of the surface texture of the imaged illuminated area of skin (2) on the basis of the difference between estimated and actual levels of light in said second waveband returned by the illuminated area of skin (2) as indicated by said received data.

18. A recording medium (7,8) in accordance with claim 17 wherein said at least one chromophore in the skin comprises melanin.

19. A recording medium (7,8) in accordance with claim 17, further storing computer interpretable instructions for causing a programmable computer (6) to be configured to receive image data representing a measurement of light returned by an illuminated area of skin (2) in a third waveband obtained by a detector (1,5), wherein the measured light in the third waveband is light having a different polarity to the light with which said area of skin (2) is illuminated to:
process (S3-2-S3-3) the image data representing measurements of light in said first and third wavebands to determine an estimated expected level of light in said second waveband returned by an illuminated area of skin utilising a model of the interaction of light with a first and a second chromophore in the skin.

20. A recording medium (7,8) in accordance with claim 19, wherein said a first and a second chromophore comprise melanin and haemoglobin.

21. A recording medium (7,8) in accordance with any of claims 17 - 20 comprising a computer disc (7).

22. A computer disc (7) in accordance with claim 21 comprising a magnetic, optical or magneto-optical disc.

23. A recording medium (7,8) in accordance with any of claims 17 - 20 comprising a signal (8) in a communications network.

## Patentansprüche

1. Methode zum Messen der Hautoberflächentextur, umfassend:
das Beleuchten eines Hautbereichs (2) mit polarisiertem Licht;
das Erhalten (S3-1) von Bilddaten, die einen Messwert von Licht darstellen, das durch den beleuchteten Hautbereich (2) in einem ersten und einem zweiten Wellenband zurückgeworfen wird, wobei das gemessene Licht in dem ersten Wellenband Licht ist, das eine andere Polarität als das Licht aufweist, mit dem der Hautbereich (2) beleuchtet wird und das gemessene Licht in dem zweiten Wellenband Licht umfasst, das dieselben und andere Polaritäten aufweist als das Licht, mit dem der Hautbereich (2) beleuchtet wird;
das Verarbeiten (S3-2-S3-3) der Bilddaten, die den Messwert von Licht im ersten Wellenband darstellen, um ein schätzungsweises erwartetes Niveau an Licht in dem zweiten Wellenband, das durch den beleuchteten Hautbereich (2) zurückgeworfen wird, unter Anwendung eines Modells der Wechselwirkung von Licht mit mindestens einem Chromophor in der Haut zu bestimmen; und
das Bestimmen (S3-4) eines Messwerts der Oberflächentextur des abgebildeten beleuchteten Hautbereichs (2) auf der Basis des Unterschieds zwischen den schätzungsweisen und tatsächlichen Niveaus an Licht in dem zweiten Wellenband, das von dem beleuchteten Hautbereich (2) zurückgeworfen wird.

2. Methode nach Anspruch 1, wobei das erste Wellenband ein Wellenband umfasst, das sichtbarem Licht entspricht.

3. Methode nach Anspruch 2, wobei das erste Wellenband ein Wellenband umfasst, das Rotlicht entspricht.

4. Methode nach Anspruch 1, 2 oder 3, wobei das zweite Wellenband ein Wellenband umfasst, das Infrarotlicht entspricht.

5. Methode nach einem der vorhergehenden Ansprüche, wobei das mindestens eine Chromophor in der Haut Melanin umfasst.

6. Methode nach einem der vorhergehenden Ansprüche, des Weiteren umfassend:
das Erhalten (S3-1) von Bilddaten, die einen Messwert von Licht darstellen, das durch den beleuchteten Hautbereich (2) in einem dritten Wellenband zurückgeworfen wird, wobei das gemessene Licht in dem dritten Wellenband Licht ist, das eine andere Polarität als das Licht aufweist, mit dem der Hautbereich (2) beleuchtet wird;
wobei das Verarbeiten (S3-2-S3-3) von Bilddaten, die den ersten Messwert von Licht darstellen, um ein schätzungsweises Niveaus von Licht in dem zweiten Wellenband, das von dem beleuchteten Hautbereich (2) zurückgeworfen wird, zu bestimmen, Folgendes umfasst:
das Verarbeiten (S3-2-S3-3) von Bilddaten, die die Messwerte von Licht in den ersten und dritten Wellenbändern darstellen, um ein schätzungsweise erwartetes Niveau von Licht in dem zweiten Wellenband, das von dem beleuchteten Hautbereich zurückgeworfen wird, unter Anwendung eines Modells der Wechselwirkung von Licht mit einem ersten und einem zweiten Chromophor in der Haut zu bestimmen.

7. Methode nach Anspruch 6, wobei das erste und zweite Chromophor Melanin und Hämoglobin umfassen.

8. Apparat zum Messen einer Hautoberflächentextur, wobei der Apparat Folgendes umfasst:
eine Lichtquelle, (3, 4), die funktionsfähig ist, um einen Hautbereich (2) mit polarisiertem Licht zu beleuchten;
einen Detektor (1, 5), der funktionsfähig ist, um:
Bilddaten zu erhalten, die einen Messwert von Licht darstellen, der von einem beleuchteten Hautbereich (2) in einem ersten Wellenband und einem zweiten Wellenband zuruckgeworfen wird, wobei das gemessene Licht in dem ersten Wellenband Licht ist, das eine andere Polarität aufweist als das Licht, mit dem der Hautbereich (2) durch die Lichtquelle (3, 4) beleuchtet wird und das gemessene Licht in dem zweiten Wellenband Licht umfasst, das dieselbe und andere Polaritäten von Licht umfasst wie das Licht, mit dem der Hautbereich durch die Lichtquelle (3, 4) beleuchtet wird; und
einen Prozessor (6, 9), der funktionsfähig ist, um:
erhaltene Bilddaten, die einen Messwert von Licht in einem ersten Wellenband darstellen, zu verarbeiten, um ein schätzungsweises erwartetes Niveau an Licht in einem zweiten Wellenband, das von einem beleuchteten Hautbereich zurückgeworfen wird, unter Anwendung eines Modells der Wechselwirkung von Licht mit mindestens einem Chromophor in der Haut zu bestimmen; und
einen Messwert der Oberflächentextur eines abgebildeten beleuchteten Hautbereichs (2) auf der Basis des Unterschieds zwischen schätzungsweisen und erhaltenen tatsächlichen Niveaus an Licht in dem zweiten Wellenband zu bestimmen, das von einem beleuchteten Hautbereich (2) zurückgeworfen wird.

9. Apparat nach Anspruch 8, wobei die Lichtquelle (3, 4), die funktionsfähig ist, um einen Hautbereich (2) mit polarisiertem Licht zu beleuchten, eine Lichtquelle (3) umfasst, die funktionsfähig ist, um einen Hautbereich (2) über ein Polarisationsfilter (4) zu beleuchten.

10. Apparat nach Anspruch 8 oder 9, wobei der Detektor (1, 5) Folgendes umfasst:
eine Digitalkamera (1), die funktionsfähig ist, um ein Bild von einem beleuchteten Hautbereich (2) über ein Polarisationsfilter (5) zu erhalten, wobei das Polarisationsfilter (5) funktionsfähig ist, um Licht in dem ersten Wellenband zu polarisieren, ohne Licht im zweiten Wellenband zu polarisieren.

11. Apparat nach Anspruch 8, 9 oder 10, wobei das erste Wellenband ein Wellenband umfasst, das sich sichtbarem Licht entspricht.

12. Apparat nach Anspruch 11, wobei das erste Wellenband ein Wellenband umfasst, das Rotlicht entspricht.

13. Apparat nach einem der Ansprüche 8 - 12, wobei das zweite Wellenband ein Wellenband umfasst, das Infrarotlicht entspricht.

14. Apparat nach einem der Ansprüche 8 - 13, wobei der Prozessor (6,9) funktionsfähig ist, um:
einen erhaltenen Messwert von Licht in einem ersten Wellenband zu verarbeiten, um einen erwarteten Wert von Licht in dem zweiten Wellenband, das von dem beleuchteten Hautbereich (2) zurückgeworfen wird, unter Anwendung eines Modells der Wechselwirkung von Licht mit Melanin in der Haut zu bestimmen.

15. Apparat nach einem der Ansprüche 8 - 12, wobei der Detektor (1, 5) funktionsfähig ist, um Bilddaten, die einen Messwert von Licht in einem dritten Wellenband darstellen, das durch einen beleuchteten Hautbereich (2) zurückgeworfen wird, zu erhalten, wobei das gemessene Licht in dem dritten Wellenband Licht ist, das eine andere Polarität als das Licht aufweist, mit dem der Hautbereich (2) beleuchtet wird; und
wobei der Prozessor (6, 9) funktionsfähig ist, um:
erhaltene Bilddaten, die Messwerte von Licht in den ersten und dritten Wellenbändern darstellen, zu verarbeiten, um ein schätzungsweises erwartetes Niveau von Licht in dem zweiten Wellenband, das von einem beleuchteten Hautbereich (2) zurückgeworfen wird, unter Anwendung eines Modells der Wechselwirkung von Licht mit einem ersten und einem zweiten Chromophor in der Haut zu bestimmen.

16. Apparat nach Anspruch 15, wobei das erste und zweite Chromophor Melanin und Hämoglobin umfassen.

17. Aufzeichnungsmedium (7, 8), das computerinterpretierbare Anweisungen speichert zum Verursachen, dass ein programmierbarer Computer (6) konfiguriert wird, um Bilddaten, die einen Messwert von Licht darstellen, das von einem beleuchteten Hautbereich (2) in einem ersten und einem zweiten Wellenband zurückgeworfen und durch einen Detektor (1, 5) erhalten wird, aufzunehmen, wobei das gemessene Licht in dem ersten Wellenband Licht ist, das eine andere Polarität aufweist als das Licht, mit dem der Hautbereich (2) beleuchtet wird und das gemessene Licht in dem zweiten Wellenband Licht umfasst, das dieselbe und verschiedene Polaritäten von Licht aufweist als das Licht, mit der der Hautbereich (2) beleuchtet wird, um:
die erhaltenen Bilddaten, die einen Messwert von Licht in der ersten Wellenbande darstellen, zu verarbeiten (S3-2-S3-3), um ein schätzungsweises erwartetes Niveau an Licht in dem zweiten Wellenband, das von dem beleuchteten Hautbereich (2) zurückgeworfen wird, unter Anwendung eines Modells der Wechselwirkung von Licht mit mindestens einem Chromophor in der Haut zu bestimmen; und
einen Messwert der Oberflächentextur des abgebildeten beleuchteten Hautbereichs (2) auf der Basis des Unterschieds zwischen schätzungsweisen und tatsächlichen Niveaus an Licht in dem zweiten Wellenband zu bestimmen (S3-4), das durch den beleuchteten Hautbereich (2), wie durch die erhaltenen Daten angezeigt, zurückgeworfen wird.

18. Aufzeichnungsmedium (7, 8) nach Anspruch 17, wobei das mindestens eine Chromophor in der Haut Melanin umfasst.

19. Aufzeichnungsmedium (7, 8) nach Anspruch 17, das des Weiteren computerinterpretierbare Anweisungen speichert zum Verursachen, dass ein programmierbarer Computer (6) konfiguriert wird, um Bilddaten, die einen Messwert von Licht darstellen, das von einem beleuchteten Hautbereich (2) in einem dritten Wellenband zurückgeworfen und durch einen Detektor (1, 5) erhalten wird, aufzunehmen, wobei das gemessene Licht in dem dritten Wellenband Licht ist, das eine andere Polarität aufweist als das Licht, mit dem der Hautbereich (2) beleuchtet wird, um
die erhaltenen Bilddaten, die einen Messwert von Licht in den ersten und dritten Wellenbändern darstellen, zu verarbeiten (S3-2-S3-3), um ein schätzungsweises erwartetes Niveau an Licht in dem zweiten Wellenband, das von einem beleuchteten Hautbereich zurückgeworfen wird, unter Anwendung eines Modells der Wechselwirkung von Licht mit einem ersten und einem zweiten Chromophor in der Haut zu bestimmen,

20. Aufzeichnungsmedium (7, 8) nach Anspruch 19, wobei ein erstes und ein zweites Chromophor Melanin und Hämoglobin umfassen.

21. Aufzeichnungsmedium (7, 8) nach einem der Ansprüche 17 - 20, eine Computerdatenscheibe (7) umfassend.

22. Computerdatenscheibe (7) nach Anspruch 21, umfassend eine magnetische, optische oder magnetooptische Datenscheibe.

23. Aufzeichnungsmedium (7, 8) nach einem der Ansprüche 17 - 20, umfassend ein Signal (8) in einem Kommunikationsnetzwerk.

## Revendications

1. Une méthode de mesure de la texture de surface cutanée, comprenant :
l'éclairage d'une zone de la peau (2) avec une lumière polarisée ;
l'obtention (S3-1) de données d'image représentant une mesure de la lumière renvoyée par la zone éclairée de la peau (2) dans une première et une deuxième longueur d'onde, dans laquelle la lumière mesurée dans la première longueur d'onde est une lumière à polarité différente de la lumière avec laquelle on éclaire ladite zone de la peau (2), et la lumière mesurée dans la deuxième longueur d'onde comprend une lumière présentant des polarités de lumière identiques à et différentes de celles de la lumière avec laquelle on éclaire ladite zone éclairée de la peau (2) ;
le traitement (S3-2-S3-3) de données d'image représentant la mesure de la lumière dans la première longueur d'onde pour déterminer un niveau de lumière prévu estimé dans la deuxième longueur d'onde renvoyée par la zone éclairée de la peau (2) éclairée, en utilisant un modèle de l'interaction de la lumière avec au minimum un chromophore dans la peau ; et
la détermination (S3-4) d'une mesure de la texture de la surface de la zone éclairée de la peau (2) imagée sur la base de la différence entre les niveaux estimés et effectifs de la lumière dans ladite deuxième longueur d'onde renvoyée par la zone éclairée de la peau (2).

2. Une méthode conforme à la revendication 1, dans laquelle ladite première longueur d'onde comprend une longueur d'onde correspondant à la lumière visible.

3. Une méthode conforme à la revendication 2, dans laquelle ladite première longueur d'onde comprend une longueur d'onde correspondant à la lumière rouge.

4. Une méthode conforme aux revendications 1, 2 ou 3 dans laquelle ladite deuxième longueur d'onde comprend une longueur d'onde correspondant la lumière infrarouge.

5. Une méthode conforme à une quelconque des revendications précédentes, dans laquelle au moins un chromophore dans la peau comprend de la mélanine.

6. Une méthode conforme à une quelconque des revendications précédentes, comprenant également :
l'obtention (S3-1) de données d'image représentant une mesure de la lumière renvoyée par la zone éclairée de la peau (2) dans une troisième longueur d'onde, dans laquelle la lumière mesurée dans la troisième longueur d'onde est une lumière à polarité différente de la lumière avec laquelle ladite zone de la peau (2) est éclairée ;
dans laquelle le traitement (S3-2-S3-3) des données d'image représentant la première mesure de la lumière pour la détermination du niveau estimé de lumière dans ladite deuxième longueur d'onde renvoyée par la zone éclairée de la peau (2) comprend :
le traitement (S3-2-S3-3) des données d'image représentant les mesures de la lumière dans lesdites première et troisième longueurs d'onde pour la détermination d'un niveau prévu estimé de lumière dans ladite deuxième longueur d'onde, renvoyée par la zone éclairée de la peau, en utilisant un modèle de l'interaction de la lumière avec un premier et un deuxième chromophore dans la peau.

7. Une méthode conforme à la revendication 6, dans laquelle lesdits premier et deuxième chromophore comprennent de la mélanine et de l'hémoglobine.

8. Un appareil de mesure de la texture de la peau, comprenant :
une source de lumière (3, 4) pouvant être utilisée pour éclairer une zone de la peau (2) à l'aide d'une lumière polarisée ;
un détecteur (1,5) pouvant être actionné pour :
obtenir des données d'image représentant la mesure de la lumière renvoyée par une zone éclairée de la peau (2) dans une première et une deuxième longueur d'onde, dans laquelle la lumière mesurée dans la première longueur d'onde est une lumière dont la polarité est différente de celle de la lumière avec laquelle ladite zone de la peau (2) est éclairée par ladite source de lumière (3, 4), et la lumière mesurée dans la deuxième longueur d'onde comprend de la lumière ayant des polarités de lumière identiques et différentes à celles de la lumière avec laquelle ladite zone de la peau est éclairée par ladite source de lumière (3, 4) ; et
un processeur (6, 9) pouvant être actionné pour :
assurer le traitement des données d'image représentant une mesure de la lumière dans une première longueur d'onde pour déterminer un niveau prévu estimé de lumière dans une deuxième longueur d'onde renvoyée par une zone éclairée de la peau, en utilisant un modèle de l'interaction de la lumière avec au minimum un chromophore dans la peau ; et
déterminer une mesure de la texture de surface d'une zone éclairée de la peau (2) en fonction de la différence entre le niveau estimé et le niveau effectivement relevé de lumière dans ladite deuxième longueur d'onde, renvoyée par une zone éclairée de la peau (2).

9. Un appareil conforme à la revendication 8, dans lequel ladite source de lumière (3, 4), pouvant être actionnée pour éclairer une zone de la peau (2) avec une lumière polarisée comprend une source de lumière (3) pouvant être actionnée pour éclairer une zone de la peau (2) à travers un filtre de polarisation (4).

10. Un appareil conforme aux revendications 8 ou 9, dans lequel ledit détecteur (1, 5) comprend :
une caméra numérique (1) pouvant être utilisée pour obtenir une image d'une zone éclairée de la peau (2) à travers un filtre de polarisation (5), dans laquelle le filtre de polarisation (5) peut être utilisé pour polariser la lumière dans ladite première longueur d'onde sans polariser la lumière dans ladite deuxième longueur d'onde.

11. Un appareil conforme aux revendications 8, 9 ou 10, dans lequel ladite première longueur d'onde comprend une longueur d'onde correspondant à la lumière visible.

12. Un appareil conforme à la revendication 11, dans lequel ladite première longueur d'onde comprend une longueur d'onde correspondant à la lumière rouge.

13. Un appareil conforme à une quelconque des revendications 8 à 12, dans lequel ladite deuxième longueur d'onde comprend une longueur d'onde correspondant à la lumière infrarouge.

14. Un appareil conforme à une quelconque des revendications 8 à 13, dans lequel ledit processeur (6, 9) peut être actionné pour :
traiter une mesure de la lumière dans une première longueur d'onde pour déterminer une valeur prévue de la lumière dans ladite deuxième longueur d'onde renvoyée par la zone éclairée de la peau (2), en utilisant un modèle de l'interaction de la lumière avec la mélanine dans la peau.

15. Un appareil conforme à une quelconque des revendications 8 à 12, dans lequel ledit détecteur (1, 5) peut être actionné pour obtenir des données d'image représentant une mesure de la lumière dans une troisième longueur d'onde renvoyée par une zone éclairée de la peau (2), dans laquelle la lumière mesurée dans la troisième longueur d'onde est une lumière ayant une polarité différente de la lumière avec laquelle ladite zone de la peau (2) est éclairée ; et
dans lequel ledit processeur (6,9) peut être utilisé pour :
traiter les données d'image représentant des mesures de la lumière dans lesdites première et troisième longueurs d'onde pour déterminer un niveau prévu estimé de la lumière dans ladite deuxième longueur d'onde renvoyée par une zone éclairée de la peau, en utilisant un modèle de l'interaction de la lumière avec un premier et un deuxième chromophores dans la peau.

16. Un appareil conforme à la revendication 15, dans lequel lesdits premier et deuxième chromophores comprennent de la mélanine et de l'hémoglobine.

17. Un dispositif d'enregistrement (7,8) mémorisant des instructions interprétables par ordinateur pour déterminer la configuration d'un ordinateur programmable (6) pour recevoir une mesure de la lumière renvoyée par une zone éclairée de la peau (2) dans une première et une deuxième longueur d'onde obtenue par un détecteur (1,5) dans lequel la lumière mesurée dans la première longueur d'onde est une lumière dont la polarité est différente de celle de la lumière avec laquelle ladite zone de la peau (2) est éclairée, et la lumière mesurée dans la deuxième longueur d'onde présente des polarités de lumière identiques à et différentes de celles de la lumière avec laquelle ladite zone de la peau (2) est éclairée pour :
traiter (S3-2-S3-3) les données d'image reçues représentant une mesure de la lumière dans la première longueur d'onde pour déterminer un niveau prévu estimé de lumière dans ladite deuxième longueur d'onde renvoyée par la zone éclairée de la peau (2), en utilisant un modèle de l'interaction de la lumière avec un minimum d'un chromophore dans la peau ; et
déterminer (S3-4) une mesure de la texture superficielle de la zone éclairée de la peau (2) imagée en fonction de la différence entre les niveaux estimés et effectifs de lumière dans ladite deuxième longueur d'onde renvoyée par la zone éclairée de la peau (2), de la façon indiquée par lesdites donnes reçues.

18. Un support d'enregistrement (7,8) conforme à la revendication 17, dans lequel au minimum un chromophores dans la peau comprend de la mélanine.

19. Un support d'enregistrement (7,8) conforme à la revendication 17, mémorisant en outre des instructions interprétables par ordinateur pour déterminer la configuration d'un ordinateur programmable (6) de façon à recevoir des données d'image représentant une mesure de la lumière renvoyée par une zone éclairée de la peau (2) dans une troisième longueur d'onde obtenue par un détecteur (1,5), dans lequel la lumière mesurée dans la troisième longueur d'onde est une lumière dont la polarité est différente de celle de la lumière avec laquelle ladite zone de la peau (2) est éclairée, pour :
traiter (S3-2-S3-3) les données d'image reçues représentant une mesure de la lumière dans lesdites première et troisième longueurs d'onde pour déterminer un niveau prévu estimé de lumière dans ladite deuxième longueur d'onde renvoyée par la zone éclairée de la peau (2), en utilisant un modèle de l'interaction de la lumière avec au minimum un premier et un deuxième chromophores dans la peau.

20. Un support d'enregistrement (7,8) conforme à la revendication 19, dans lequel un premier et un deuxième chromophores comprennent de la mélanine et de l'hémoglobine.

21. Un support d'enregistrement (7,8) conforme à une quelconque des revendications 17 - 20, comprenant un disque d'ordinateur (7).

22. Un disque d'ordinateur (7) conforme à la revendication 21, comprenant un disque magnétique, optique ou magnéto-optique.

23. Un support d'enregistrement (7,8) conforme à une quelconque des revendications 17 - 20, comprenant un signal (8) dans un réseau de communications.
